# EUROPEAN PATENT SPECIFICATION

(11) **EP 2 575 703 B1**
(45) Date of publication and mention of the grant of the patent: **29.07.2015**
(21) Application number: 11777798.7
(22) Date of filing: 13.04.2011
(51) Int. Cl.: A61F 5/44

(54) **DRAINAGE APPLIANCE FOR BODY WASTE**
DRAINAGEVORRICHTUNG FÜR KÖRPERABFÄLLE
APPAREIL DE DRAINAGE POUR DÉCHETS CORPORELS

(30) Priority: 18.06.2010 US 356072 P; 03.05.2010 US 330506 P
(43) Date of publication of application: 10.04.2013
(73) Proprietor: ConvaTec Technologies Inc., Las Vegas, NV 89169-6754 (US)
(72) Inventor: GREGORY, Christopher, Skillman,New Jersey 08558 (US)
(74) Representative: Mays, Julie
(86) International application number: PCT/US2011/032244
(87) International publication number: WO 2011/139498

(56) References cited:
- EP-A2- 1 514 572
- WO-A1-2008/157172
- US-A- 4 344 434
- US-A- 5 078 679
- US-A- 6 132 397
- US-A1- 2005 054 996
- US-A1- 2008 177 232
- US-A1- 2008 312 614

## Description

### Field of the Invention

The present invention relates to an indwelling drainage appliance for draining body waste from the rectum, intestinal or urinary tract. The invention is especially, but not exclusively, useful for an appliance in the form of a drainage catheter.

### Background to the Invention

US patent application US2005/0054996A1 discloses a waste management appliance for the rectum.

Indwelling fecal management catheters are utilized to capture and contain liquid or semi-liquid fecal matter of non-ambulatory hospital patients. This has multi-fold benefits including: the prevention of contamination of a patient's skin by corrosive effluent; the reduction of risk of contamination by potentially infectious material; and minimization of soiling of the bedding. Most fecal management catheters in use include an annular inflatable retention balloon cuff surrounding the distal end of the catheter. The cuff serves to: (i) rest against the floor of the rectal vault above the external sphincter to prevent the catheter from being expelled, and (ii) effect a seal to the rectum, to ensure that effluent is diverted into the catheter as the only exit for the effluent. The effectiveness in draining waste from the body is dependent on correct inflation of the cuff. The inflation pressure should be sufficiently high to retain the appliance properly seated, and provide the desired seal against the internal tissue. Occasionally, the cuff may be insufficiently inflated and the appliance becomes dislodged from its position, resulting in ineffective waste management. There are several reasons for insufficient inflation, including loss of fluid due to leaks; shifting of placement within the body; relaxation of the local anatomy; and permeation of fluid through the material of the balloon cuff wall. In addition to the concerns about insufficient inflation of the cuff, it is equally important to avoid over-inflation, because sustained application of high pressure on the surrounding anatomy may affect local tissues, for example, by restricting blood perfusion within the tissue or stimulate a defication response ejecting the device..

US-A-2005/0054996 describes one example of a drainage catheter with an inflatable cuff. The cuff is pre-formed in its fully inflated shape and size, to enable full inflation with just modest inflation pressure. A syringe is used to supply inflation fluid to inflate the cuff. Two fundamentally different, alternative techniques are described for controlling maintenance of inflation pressure in the cuff after inflation:
(i) the inflation system can allow the balloon cuff to shrink in volume if the interface pressure between the device and the internal tissue increases, maintaining only the desired pressure on the tissue, such as by spring-loading the syringe plunger to a predetermined level. The syringe remains in open fluid communication with the cuff, to maintain self-adjustment of the cuff;
(ii) the balloon cuff can be fixed in volume once the pressure is reached on insertion and initial inflation. The inflation line can be sealed closed by a valve. If this is done at a time when the bowel is relaxed, the pressure on tissue will only increase when the bowel is in constriction. It is said that, as this is only a periodic event, the tissue will be fully perfused between constrictions.

It would be desirable to further enhance the techniques for maintaining a cuff in a good seating and sealing state, without sustained application of excessive pressure to surrounding tissue.

### Summary of the Invention

The invention provides an appliance for draining body waste according to claim 1. Further embodiments of the invention are defined in the dependent claims.

The following presents a simplified summary of the invention in order to provide a basic understanding of some aspects of the invention. This summary is not an extensive overview of the invention. It is intended to neither identify key or critical elements of the invention nor delineate the scope of the invention. Its sole purpose is to present some concepts of the invention in a simplified form as a prelude to the more detailed description that is presented later.

Broadly speaking, one aspect of the invention controls cuff distension by a combination of:
(a) a resiliently deformable device for urging distension of the cuff, the resiliently deformable device being configured to deform resiliently when the cuff is compressed, the resilient deformation generating a return force to counter the compression and urge distension;
(b) a fluid-containing chamber of variable size depending on at least one selected from: degree of distension of the cuff; degree of deformation of the resiliently deformable device;
(c) a fluid-transfer damper for restricting fluid flow with respect to the chamber when the chamber changes size, at least when the change corresponds to compression of the cuff.

With such an arrangement, at steady state equilibrium, the pressure exerted by the cuff on surrounding body tissue in the rectum or urinary tract depends on the degree of deformation of the resiliently deformable device. The pressure characteristic is set by parameters including, for example, the spring modulus and/or size of the resiliently deformable device. The body tissue exerts a natural counter force, tending to compress the cuff, and a balance is achieved where the cuff adopts a distended size appropriate for the cavity in the rectum, intestinal or urinary tract (inclusive of the bladder) and the pressure exerted between the cuff and the tissue is balanced by the resilient deformation of the resiliently deformable device.

The fluid-containing chamber and the fluid-transfer damper act to restrict the rate at which the cuff can change volume, by generating a dynamic resistance at least to compression. Compression of the cuff results, directly or indirectly, in a change of volume of the chamber, producing a back pressure (positive or negative) in the fluid within the chamber that directly or indirectly resists the compression. Fluid flows through the damper to relieve the back pressure, but the damper restricts the flow of fluid such that the back pressure subsides only relatively slowly. The result is that the cuff compresses more slowly than would be allowed by the resiliently deformable device alone. The fluid-transfer damper may act also to restrict the rate at which the cuff can change volume during distension, or the fluid-transfer damper may be configured substantially not to hinder distension, only compression.

The above techniques can provide enhanced control of the cuff, with a characteristic including both (i) self-adjustment of the cuff over time, and (ii) resistance to rapid compression of the cuff. The self-adjustment of the cuff over time enables the cuff to compensate automatically for changes in anatomical volume for example if the wearer changes position, and maintain correct distension, without sustained application of excess pressure. If the cuff is of a fluid-inflated type, the characteristic also permits compensation for small fluid leakage, for example, permeation through the cuff wall material, or leakage through small imperfections. At the same time, the resistance to rapid compression enables the cuff to withstand transient anatomical pressure perturbations without the cuff collapsing. This permits the cuff to resist short-term pressure increases, such as pressure from peristalsis, or as may occur should the wearer cough. Such pressure increases might otherwise cause the resiliently deformable device to reduce cuff distension in order to compensate for apparent "over-pressure". Resisting rapid compression maintains cuff distension, thereby preventing the cuff collapsing under the temporary increase in pressure, and maintaining the cuff correctly seated in the rectum, intestinal or urinary tract. As an alternative to the fluid damping being done by a passive flow limiting feature, an active configuration could be used with a sensor monitoring the pressure in the cuff and a timer delaying the release of pressure for a predetermined time. The release of excess pressure could be done with an electrically driven valve.

The resiliently deformable device may comprise at least one resiliently deformable member. The resiliently deformable device may be configured to be resiliently compressible, or resiliently expandable, according to the design preferred. Some example embodiments use resiliently deformable foam, for example, resiliently compressible foam. Other example embodiments use a resiliently deformable spring. Other example embodiments use a resiliently compressible foam that is nearly closed cell so that the fluid within can only escape from the foam slowly thus the foam itself becomes the fluid dampening mechanism by limiting flow.

In some embodiments, the resiliently deformable device is disposed within the cuff to directly bear on the cuff. In other embodiments, the resiliently deformable device is spaced from the cuff, and is in pressure-communication with the cuff (for example, by inflation fluid).

In some embodiments, the fluid-containing chamber is defined at least partly by the cuff. In other embodiments, the fluid-containing chamber is distinct from the cuff, and is associated with the resiliently deformable device. In either case, the resiliently deformable device may optionally be disposed at least partly within the chamber to achieve the functional volume dependency of the chamber, but other arrangements for operatively changing the chamber volume may be used as desired. The chamber may be configured to decrease in volume for a decrease in size of the resiliently deformable device, or it may be configured to increase in volume with a decrease in size of the resiliently deformable device.

The fluid may be liquid (e.g., water or saline) or a gas (e.g., air). In the example embodiments, the fluid is air and the fluid-transfer damper communicates with the surrounding atmosphere.

Although features believed to be of importance are highlighted herein and in the appended claims, protection may be sought for any novel feature or idea described herein and/or illustrated in the drawings whether or not emphasis has been placed thereon.

### Brief Description of the Drawing:

Fig. 1 is a schematic section illustrating a fecal management catheter in a first embodiment of the invention.
Fig. 2 is a schematic section illustrating a first example of flow restriction port used in Fig. 1.
Fig. 3 is a schematic section illustrating a second example of flow restriction port used in Fig. 1.
Fig. 4 is a schematic section illustrating a modified distal portion including a bolster chamber.
Fig. 5 is a schematic section illustrating a fecal management catheter in a second embodiment of the invention similar to the first embodiment.
Fig. 6 is a schematic diagram illustrating an active fluid transfer damper.
Fig. 7 is a schematic section illustrating an intestinal catheter used in a transcecal ileostomy.

### Detailed Description of the Invention:

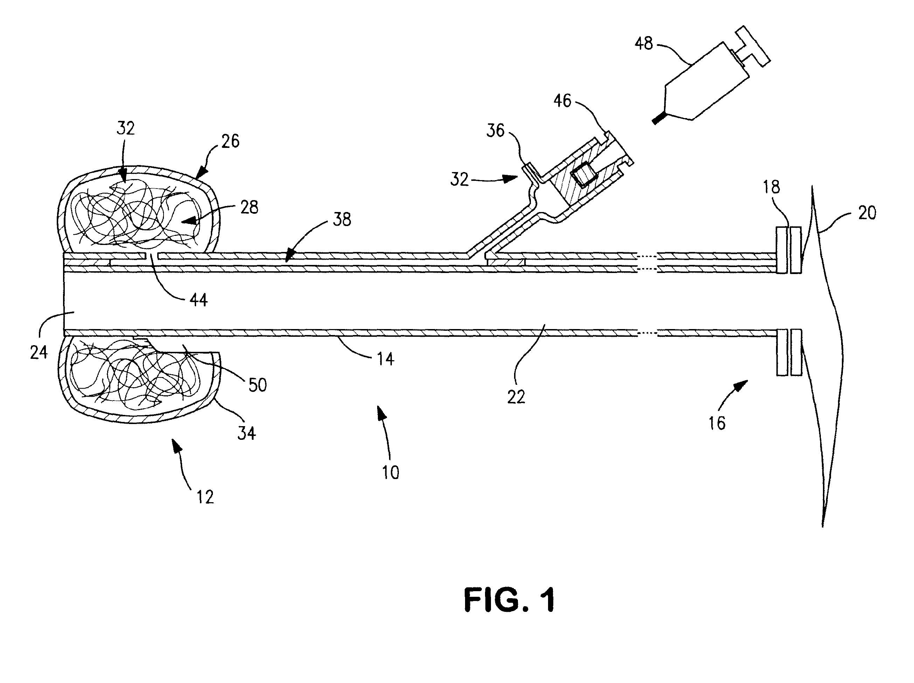

Fig. 1 illustrates generally a drainage appliance 10 having a distal portion 12 insertable into the rectum or urinary tract of a wearer to collect body waste. The dimensions of the appliance 10 are typically suited to fecal management, although it will be appreciated that the same principles may also be used for urinary management. The appliance 10 is illustrated in the form a catheter tube 14, although the appliance 10 may have any suitable form. A proximal portion 16 of the appliance 10 is coupled, or coupleable via a separable coupling 18, to an effluent collection device 20, here illustrated as a pouch. A drainage passage 22 extends in the tube 14 from the distal portion 12 to the proximal portion 16 for transporting body waste from the body to the effluent collection device 20. In the illustrated embodiments, the drainage passage 22 has a permanently open mouth 24 at the distal portion, so that body waste can enter the drainage passage 22 unvalved. This is believed to contribute to avoiding concentration of pressure on body tissue in the region of the distal portion 12. However, in other embodiments (not shown), a valve (e.g., an inflatable balloon) may be provided for selectively closing the mouth 24 to entry of effluent until evacuation is desired.

The distal portion 12 comprises a distensible cuff 26, for engaging internal body tissue when the distal portion 12 is in situ in the body. The cuff 26 extends peripherally (e.g., circumferentially) around an outer surface of the catheter tube 14 at the distal portion 12, and is such that the cuff 26, when distended, defines an outward projection or shoulder with respect to the surface of the tube 14. The cuff 26 serves to retain the distal portion in situ once inserted into the rectum, intestinal or urinary tract, and/or the cuff 26 serves to effect a seal to divert the body waste into the appliance 10 as the only exit for the body waste. In all embodiments, distension of the cuff 26 is controlled by a combination of:
(a) a resiliently deformable device 28 for urging distension of the cuff 26, the resiliently deformable device 28 being configured to deform when the cuff 26 is compressed, the resilient deformation generating a return force to counter the compression. The resilient deformation may be compression or expansion;
(b) a fluid-containing chamber 30 of variable size depending on at least one selected from: degree of distension of the cuff 26; degree of deformation of the resiliently deformable device 28. The resiliently deformable device 28 may optionally be disposed at least partly within the chamber 30;
(c) a fluid-transfer damper 32 for restricting fluid flow with respect to the chamber 30 when the chamber 30 changes size, at least when the change corresponds to compression of the cuff 26.

In the present embodiment, the resiliently deformable device 28 is configured to compress resiliently. The resiliently deformable device 28 is disposed within the cuff 26 in order to bear directly on the cuff 26 to urge distension. The chamber 30 is defined at least partly by the cuff 26. Compression of the cuff 26 resiliently compresses the resiliently deformable device 28 and reduces the volume of the chamber 30.

Also in the present embodiment, the resiliently deformable device 28 makes the cuff 26 at least partly self-distending without application of pressure from an external source of inflation fluid. Fluid is still used within the cuff 26, but as a means for providing auxiliary control over cuff distension. The resiliently deformable device 28 may have any suitable shape, such as annular, ring, or other open-loop or closed-loop shaped. The resiliently deformable device 28 generates an urging force responsive to compression of the cuff 26.

In one form (not shown), the resiliently deformable device 28 may comprise multiple segments, each radially extending, and arranged sequentially in the circumferential direction. Viewed in another way, the device 28 may comprise radial slots dividing the device into the segments. The segments may project radially from a central closed-loop hub. The segments enable the resiliently deformable device to have a high degree of conformability, because each segment deforms substantially independently of the others.

In another form, the resiliently deformable device 28 may have a spring constant that varies in the radial direction. For example, the spring constant may be greater near the centre than near the radially outer periphery. The spring constant at an inner region may be at least two times that at the outer periphery, preferably between about 4 and 8 times that at the inner periphery. The different in spring constant may be implemented in a single block of material, or the resilient device 28 may be divided into nested (e.g. concentric) pieces that together make up a composite device 28. Each piece has a different spring constant to give a desired gradient from inner to outer periphery.

The resiliently deformable device 28 may have a natural (e.g., substantially non-compressed) form that is smaller than a natural (e.g., unstressed) form of the cuff 26, or substantially the same size as the natural form of the cuff 26, or larger than the natural form of the cuff 26. The resilient device 28 may of any suitable material or materials. In a preferred form, the device 26 is of compressible foam, for example, open-cell foam. The foam may be of polyurethane (e.g., open-cell polyurethane foam), although any other resiliently compressible material(s) may be used as desired.

Preferably, the resiliently deformable device 28 is configured or selected such that, even with maximum permitted deformation, the urging pressure exerted by the cuff 26 does not exceed a predetermined desired threshold, considered appropriate for sustained application to tissue without stimulating a response.. For example, the maximum appropriate threshold may be 35 mmHg. Additionally or alternatively, the resiliently deformable device is preferably configured to be capable of operative deformation of at least 2/3 of it's original volume through elastic deformation.

The cuff 26 and the chamber 30 within may be defined at least partly by a flexible cuff wall 34 that is sealed to the exterior surface of the tube 14 to contain the resiliently deformable device 28. In one form, the cuff wall 34 is formed (e.g., molded) in a substantially fully distended form. The fully distended form is the maximum size and shape that the cuff 26 will adopt in normal use when inserted into the rectum, intestine or urethra. Alternatively, the cuff wall 34 may be formed oversize, larger than the fully distended form. An advantage of forming the cuff 26 in its fully distended form, or larger, is that the material of the cuff wall 34 does not have to stretch in order to reach the fully distended form. This enables distension of the cuff 26 at a generally lower urging pressure exerted by the resilient device 28 than if the cuff wall 34 needs to stretch elastically to reach the fully distended form. A lower urging pressure may be advantageous in reducing the risks of excessive pressure being applied to the surrounding anatomy. The cuff wall material may be elastically stretchable (even though such stretching would not occur in normal use), or substantially non-stretchable. In a further alternative form, the cuff wall 34 may be formed with a size and/or shape smaller than the fully distended form, and the cuff wall 34 being of elastically stretchable material configured to stretch under the influence of the urging force exerted by the resilient device 28. The fully distended form of the cuff 26 is then defined by a balance between the opposing restoration forces exerted by compression of the resilient device 28 and elastic stretching of the cuff wall 34.

The cuff wall 34 may be of any suitable biocompatible material, optionally elastically stretchable or substantially non-elastically-stretchable. Non-limiting example materials include polyurethane, silicone rubber, or thermoplastic elastomer. The cuff wall may, for example have a thickness of between about 5 microns and about 50 microns if made of essentially inelastic material or 100 microns to 750 microns if the material is elastic. The wall 34 may be substantially impervious to the fluid used within the chamber 30. As used herein, "substantially impervious" may mean that the flow rate of fluid through the wall 32 is less than about 1 ml/day at a pressure differential of 35 mlHg. The fluid may be a gas (e.g., air) or a liquid (e.g., saline or water). In the present embodiment, the fluid is air.

The fluid-transfer damper 32 is configured to regulate fluid flow responsive to a change in volume of the chamber 20. In the illustrated form, the fluid-transfer damper 32 is generally passive. The fluid-transfer damper 32 comprises a flow restriction 36 that impedes flow of fluid through the damper 32. In the present embodiment, the fluid-transfer damper 32 is coupled in direct communication with the chamber 30 within the cuff 26 by a fluid conduit 38 extending between the cuff 26 and the fluid-transfer damper 32, optionally located at a region of the appliance 10 that, in use, is external to the body of the wearer. In other embodiments (described later), the fluid-transfer damper 32 may be in indirect communication with the internal volume of the cuff 26, e.g. in a different fluid containing region or path than the cuff 26.

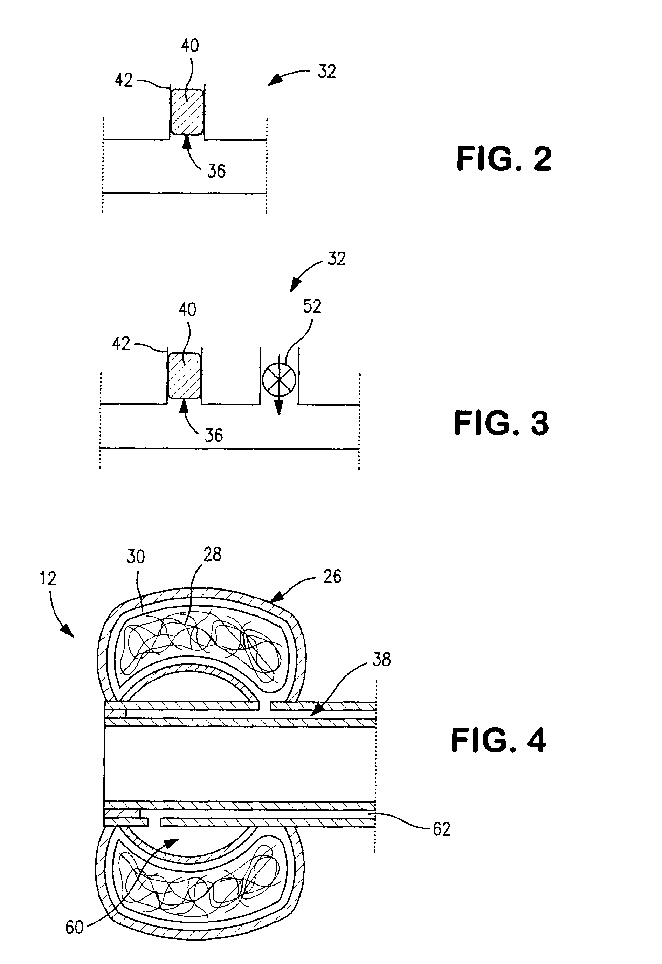

Various types of fluid-transfer damper 32 may be used. The damper 32 may have the same restriction characteristic impeding fluid flow in both directions (inlet and outlet). For example, referring to Fig. 2, the restriction 36 may comprise a microporous plug 40 fitted to a port 42. One side of the port 42 communicates with the conduit 38, and the other side of the port 42 communicates with ambient atmosphere. Other forms of partially permeable plugs, membranes or other restrictions may be used as desired, for example, of PTFE, silicone rubber or dense polyurethane foam. The restriction 36 may have a flow rate characteristic that is dependent on the pressure differential across the restriction 36, such that a greater pressure differential produces a higher flow rate. Such a pressure dependency permits very large excess pressure differentials to subside more quickly. The flow rate of the restriction 36 may be in a range of about 2-15 ml/minute for a pressure differential of 30 mmHg. This may define a shrink-down time for the cuff in a range of from about 3 to about 10 minutes. The restriction 36 may define a damping time (e.g. the time period for the pressure to decay to 1/e of an initial value, where "e" is the natural constant 2.718...) that lies in a range of from about 3 to about 10 minutes. The restriction 36 therefore serves to slow at least fluid out-flow from the chamber 30, in order to resist rapid compression of the cuff 26.

In the illustrated form, the fluid transfer conduit 38 extends at least partly within the tube 14, and is optionally integrated with, or attached to, the wall of the tube 14.

At the distal portion 12, the fluid transfer conduit 38 communicates, via an aperture 44, with the chamber 30 within the cuff 26. At the opposite end, the fluid transfer conduit 38 is coupled to the fluid-transfer damper 32 and, in parallel, to a connector 46 for an external deflation/inflation source 48. The connector 46 may, for example, be a Luer activated valve port. The parallel arrangement means that the fluid-transfer damper 32 does not interfere with fluid admission/withdrawal through the connector 46. The source 48 may, for example, be a syringe or other manual or automatic pump. If desired, instead of the source 48 being an external device coupled by the connector 46, the source 48 may be integrated as part of the appliance 10, without or without the connector 46.

In use, in order to prepare the appliance 10 for insertion into the rectum, intestinal or urinary tract, the source 48 (e.g., syringe) is used to withdraw fluid from the chamber 30 within the cuff 26 via the fluid transfer conduit 38. Withdrawal of fluid creates negative pressure in the cuff 26 with respect to the external ambient atmosphere, and the surrounding atmospheric pressure collapses the cuff 26 and the resiliently compressible device 28 within. The fluid-transfer damper 32 will slowly leak fluid (air) back into the appliance 10, but only relatively slowly, providing ample time for insertion of the appliance 10 into its operative position while the cuff 26 remains collapsed.

With the cuff 26 collapsed, the distal portion 12 is inserted into the patient's rectum, intestinal or urinary tract as desired for the appliance 10. In one form, a finger pocket or recess 50 is provided between a portion of the cuff 26 and the tube 14 for receiving a fingertip to aid guidance and insertion of the distal portion 12 into position. Once inserted, the source 48 (e.g., syringe) is used to re-inject the fluid via the fluid conduit 36, allowing the cuff 26 to distend. In general, the cuff 26 is not maintained distended by positive inflation pressure within the cuff 26, but instead is self-distending by the action of the resiliently deformable device 28. Re-injecting the fluid allows restoration of atmospheric fluid pressure in the cuff, enabling the resiliently deformable device to decompress. However, it will also be appreciated that, upon insertion into the rectum or urinary tract, the cuff 26 may be expected to distend to a slightly smaller size in situ, than the fully distended size prior to use. Reinjecting the same quantity of fluid might generate a slight positive fluid pressure within the cuff 26, but any such positive pressure will not be sustained, as it will progressively diminish as the excess fluid escapes slowly through the fluid-transfer damper 32 to restore atmospheric fluid-pressure equilibrium, as explained below. Temporary positive pressure during the insertion process can help to move tissue or fecal matter out of the way of the device allowing it to position properly.

The process of coming to equilibrium involves the flow of fluid through the fluid-transfer damper 32. The pressure on tissue is ultimately determined by the spring modulus and/or size of the resiliently deformable device 28 within the cuff 26. If the cuff 26 is over-distended, the pressure exerted between the cuff 26 and the tissue is higher than the spring modulus of the resiliently deformable device 28 is designed to bear. The cuff 26 is squeezed, and the fluid within the chamber 30 comes under pressure. The pressure (or back-pressure) in the fluid is substantially equal to the excess pressure that the resiliently deformable device 28 does not bear. The back-pressure resists compression of the cuff 26. Fluid from the chamber 30 passes outwardly through the fluid-transfer damper 32 to relieve the back-pressure, but the fluid-transfer damper 32 restricts the fluid flow, such that the back-pressure subsides slowly. The flow rate through the damper 32 is dependent on the pressure differential, in order to avoid a high excessive pressure being maintained for too long. However, the cuff 26 compresses progressively at a slower rate than would be allowed by the resiliently compressible device 28 alone. As the cuff 26 compresses, the resiliently deformable device 28 deforms further, thereby generating a progressively increasing urging force to counter the compression. The fluid continues to escape until the fluid pressure in the chamber 30 is atmospheric (zero back-pressure), at which point the balance is restored between the pressure exerted between the cuff 26 and the body tissue, and the resilient deformation of the device 28.

If the cuff 26 is under-distended, and the expansion pressure generated by the resiliently deformable device 28 is greater than tissue resistance, fluid will be drawn in through the fluid-transfer damper 32, and the resiliently deformable device 28 will tend to expand the cuff 26 to the fully distended form, or until tissue resistance matches the modulus of the resiliently deformable device 28. As explained above, if the fluid-transfer damper 32 also restricts the rate of fluid in-flow, the cuff 26 will tend to distend more slowly than under the influence of the resiliently deformable device 28 alone. Such a characteristic provides a certain delay that permits the cuff 26, when compressed, to be inserted into the rectum, intestinal or urinary tract.

With such an arrangement, at steady state, the pressure exerted by the cuff 26 on surrounding body tissue in the rectum, intestinal or urinary tract depends on the degree of deformation of the resiliently deformable device 28. The pressure characteristic is set by parameters including, for example, the spring modulus and/or size of the resiliently deformable device. The body tissue exerts a natural counter force, tending to compress the cuff 26, and a balance is achieved where the cuff 26 adopts a distended size appropriate for the cavity in the rectum, intestinal or urinary tract, and the pressure exerted between the cuff 26 and the tissue is balanced by the resilient deformation of the resiliently deformable device.

The fluid-containing chamber 30 and the fluid-transfer damper 32 act to restrict the rate at which the cuff can change volume, by generating a dynamic resistance at least to compression. Compression of the cuff 26 causes a change of volume of the chamber 30, producing a back pressure in the fluid within the chamber 30 that directly resists the compression. Fluid flows through the damper 32 to relieve the back pressure, but the damper 32 restricts the flow of fluid such that the back pressure subsides only relatively slowly. The result is that the cuff 26 compresses more slowly than would be allowed by the resiliently deformable device 28 alone.

The above techniques can provide enhanced control of the cuff 26, with a characteristic including both (i) self-adjustment of the cuff 26 over time, and (ii) resistance to rapid compression of the cuff 26. The self-adjustment of the cuff 26 over time enables the cuff 26 to compensate automatically for changes in anatomical volume for example if the wearer changes position, and maintain correct distension, without sustained application of excess pressure. At the same time, the resistance to rapid compression enables the cuff 26 to withstand transient anatomical pressure perturbations without the cuff 26 collapsing. This permits the cuff 26 to resist short-term pressure increases, such as pressure from peristalsis, or as may occur should the wearer cough. Such pressure increases might otherwise cause the resiliently deformable device 28 to reduce cuff distension in order to compensate for apparent "over-pressure". Resisting rapid compression maintains cuff distension, thereby preventing the cuff 26 collapsing under the temporary increase in pressure, and maintaining the cuff 26 correctly seated in the rectum, intestinal or urinary tract.

The fluid-transfer damper 32 may optionally include a status indicator (not shown) indicating whether a pressure differential exists at the damper 32, at least of a certain magnitude. For example, the status indicator may be an expandable element responsive to pressure differential. If a pressure differential exists, this is indicative that the cuff has not reached equilibrium, and is in the process of changing shape, or resisting short term pressure perturbations. If no pressure differential exists, this indicates that the cuff has reached equilibrium. Such an indication may be useful for practitioners and care-givers, especially to verify the status of the cuff after insertion into the rectum or urinary tract.

The example of fluid-transfer damper 32 of Fig. 2 has substantially the same restriction to fluid flow in either direction of flow of fluid through the damper 32. If preferred, the fluid-transfer damper 32 may be configured to have a directional flow characteristic (e.g., different flow characteristics for inflow an outflow directions). For example, Fig. 3 illustrates a fluid-transfer damper 32 similar to that of Fig. 2, but additionally comprising a directional bypass valve 52. The bypass valve 52 is responsive to pressure differential across the valve 52. A positive pressure on the side of the valve 52 communicating with the chamber 30 causes the valve 52 to close, such that the damper 32 restricts the flow rate of fluid flowing out of the chamber 30 in the same way as described for Fig. 2. A negative pressure on the side of the valve 52 communicating with the chamber 30 causes the valve 52 to open to bypass the restriction 36, and allow in-flow of fluid to relieve the negative pressure, at a faster flow rate than through the restriction 36. The flow rate for in-flow may be substantially unrestricted so that the speed of distension is controlled mainly by the resiliently deformable device, or the flow rate for inflow may be restricted (e.g. by the valve 52) to a lesser extent than the flow rate set by the restriction 36. For example, the flow rate for in-flow may be at least 50ml per minute (at a pressure differential of 20 mmHg). The cuff 26 may fill from a fully collapsed form to a fully distended form in a minute or less.

Fig. 4 illustrates a modified distal portion 12 of the appliance 10. The cuff 26 is partitioned into two chambers 30 and 60, one arranged outside, or around, or surrounding the other. The outer chamber is the chamber 30 containing the resiliently deformable device 28. The inner chamber is a bolster chamber 60 that provides an inflatable support or bolster for the distal portion 12. An inflation conduit 62, optionally functionally independent of the fluid conduit 28, communicates with the bolster chamber 60 to permit controlled inflation and/or deflation with a bolster inflation fluid. The bolster inflation fluid may be the same as, or different from, the fluid used within the chamber. The bolster fluid may be liquid (e.g., water or saline), or gas (e.g., air). The bolster chamber 60 is optionally made of material that, at least at the pressure used to inflate the bolster chamber 60, does not stretch substantially.

When inflated, the bolster chamber 60 provides an additional degree of support at the cuff 26. The bolster chamber 60 holds the tube 14 open at distal portion 12 ensuring that, even though the tube 14 may be made of a relatively soft and flexible material, the mouth 24 does not become substantially constricted. Even though the cuff 26 is configured to vary in distension during use, the bolster chamber 60 remains substantially inflated, and changes in cuff size are accommodated by changes in the volume of the chamber 30. The chamber 30 also acts as a self-adjusting, compliant cushion around the bolster chamber 60 so that the inflation pressure of the bolster chamber is not applied to the body tissue.

For insertion or removal of the appliance 10, the bolster fluid is evacuated from the bolster chamber 60. With the bolster chamber 60 deflated, the distal portion 12 is substantially compliant, allowing the tube 14 to be collapsed for ease of passage through the rectum, intestinal or urinary tract.

The bolster chamber 60 may be inflated by fluid from a source urged by a second resiliently deformable device (not shown) generally stiffer than the resiliently deformable device 28, in order to inflate the bolster chamber 60 to a firm pressure. Once inflated, the bolster chamber 60 may be sealed to prevent the bolster chamber collapsing in use until it is desired to collapse the bolster chamber 60 for removal of the appliance 10.

Additionally or alternatively, the provision of the bolster chamber 60 may enable the spring constant of the resiliently deformable device 28 to be softer than if the bolster chamber 60 is omitted. The bolster chamber 60 can perform some of the structural support function that would otherwise have to be performed by the resiliently deformable device 28 alone. In one form, the in order to collapse the cuff 26 to a form insertable to or removable from its operative position in the body, it is possible merely to evacuate the fluid from the bolster chamber 60. Without the bolster, the outer chamber 30 may be sufficiently soft and compliant even without evacuating its fluid. Optionally, the connector 46 may be omitted.

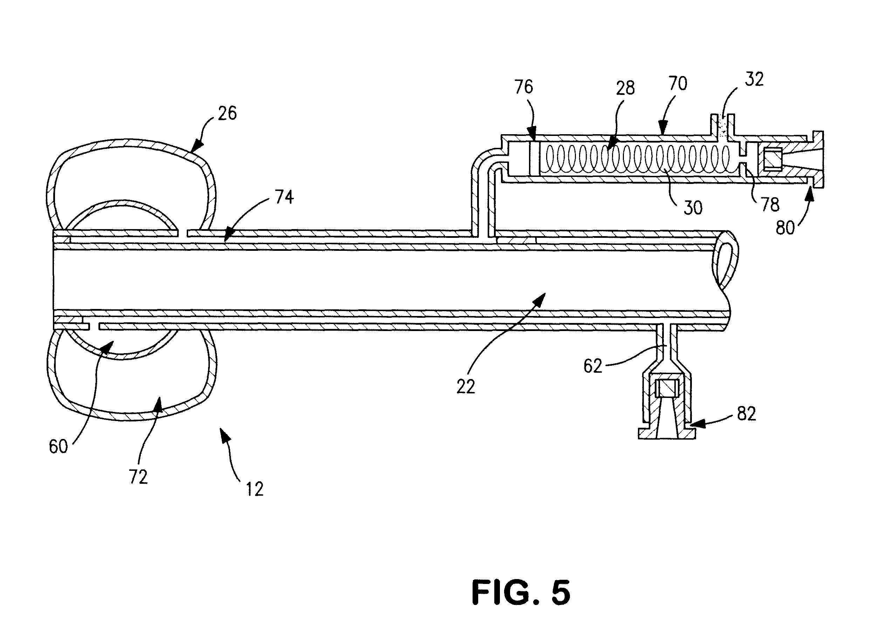

Fig. 5 illustrates a second embodiment of the invention. The second embodiment is similar to the first embodiment, and the same reference numerals denote equivalent features to those already described. The main difference is that, in the second embodiment, the resiliently deformable device 28 and the associated fluid-containing chamber 30 are arranged in a distension control unit 70 outside the cuff 26. The distension control unit 70 is in pressure communication with an inflation chamber 72 of the cuff 26 by means of an inflation line 74. The provision of the distension control unit 70 outside the cuff 26 may enable the cuff 26 to be made more compact, and collapse to a smaller shape, than in the first embodiment, but at the expense of additional fluid coupling to provide pressure communication between the cuff 26 and the distension control unit 70.

The distension control unit 70 comprises a movable seal or plunger 76 separating the chamber 30 from the inflation fluid side. One end (or portion) of the resiliently deformable device 28 is configured to be engaged by the plunger 76, and an opposite end (or other portion) of the resiliently deformable device 28 rests against a fixed seat 78. The movable plunger 76 and the inflation line 74 subject the resiliently deformable device 28 to substantially the same pressure within the cuff chamber 72, but the chamber 30 remains out of fluid communication with the cuff chamber 72. The inflation fluid used within the cuff chamber 72 may be same as, or different from, the fluid in the chamber 30. The inflation fluid may be liquid (e.g. water or saline), or a gas (e.g. air).

In the form illustrated in Fig. 5, the resiliently deformable device 28 comprises a spring, for example of metal. The spring is configured as a compression spring, although in other examples an extendable spring may be used. The spring is optionally an elongate coil spring, which can provide relatively large deformation. If preferred the spring could also be replaced by resiliently deformable foam as in the first embodiment.

The fluid-transfer damper 32 may be substantially the same as described for the first embodiment, and disposed at the distension control unit 70 to communicate with the chamber 30.

The distension control unit 70 further comprises a connector 80 similar to the connector 46 of the first embodiment. The connector 80 permits fluid to be withdrawn from the chamber 30 in order to collapse the cuff 26 for insertion or removal of the distal portion 12 of the appliance 10 with respect to the rectum, intestinal or urinary tract. Withdrawal of fluid from the chamber 30 creates a negative pressure in the chamber 30 with respect to the pressure (typically atmospheric pressure) on the inflation side communicating with the cuff 26. In effect, the atmospheric pressure acting on the cuff 26 is no longer balanced within the chamber 30. The pressure differential causes the plunger 76 to squeeze the resiliently deformable device 28 causing compression of the device 28. The inflation fluid within the cuff 26 transfers towards the distension control unit 70, allowing the cuff 26 to collapse.

The function of the second embodiment when in the body is similar to the first embodiment, except that the resiliently deformable device 28 and the chamber 30 are arranged distinct from the cuff 26, and pressure is communicated between distension control unit 70 and the cuff 26 by the inflation line 74.

At equilibrium, the fluid-transfer damper 32 plays no role. The pressure exerted between the cuff 26 and the surrounding body tissue depends on the degree of deformation of the resiliently deformable device 28, and parameters such as the spring modulus and/or size of the resiliently deformable device 28. The fluid-transfer damper functions to provide a dynamic resistance to change in deformation of the resiliently deformable device 28, at least when the change corresponds to compression of the cuff 26. In addition to the same self-adjusting effect for controlling cuff distension, the second embodiment can also compensate for any small leakage of inflation fluid from the chamber 72 of the cuff 26. Small leakage might be caused, for example, by permeation through the cuff wall material, or by leakage through small imperfections.

The degree of deformation of the resiliently deformable device 28 may provide an indication of the fill status of the cuff 26. At least a portion of the housing of the distension control unit 70 may define a window to enable the state of the resiliently deformable device 28, and/or the position of the plunger 76 to be viewed. The window may be include indicia or other calibrations to indicate the cuff fill status.

The second embodiment may also comprise the optional bolster chamber 60, and the bolster inflation line 62, for providing additional structural support at the distal portion 12 of the appliance 10. A connector 82 provides a port communicating with the bolster inflation line 62. Although not shown in Fig. 5, the second embodiment may also include a coupling 18 and effluent collector 20 similar to the fist embodiment, and/or the finger pocket 52.

Although the fluid-transfer damper 32 is illustrated in the foregoing embodiments as a separately identifiable device, the damper 32 may be implemented by, or combined with, another device. For example, the fluid-transfer damper 32 may be defined by a fluid line of limited cross-section that defines a restriction or resistance to fluid flow therethrough. In an illustrative example not forming part of the invention, the fluid-transfer damper 32 may be integrated as part of the cuff 26 or resilient deformable device 28, by again defining a restriction to fluid flow therethrough. For example, the damper may be defined by the cuff material itself restricting fluid flow therethrough.

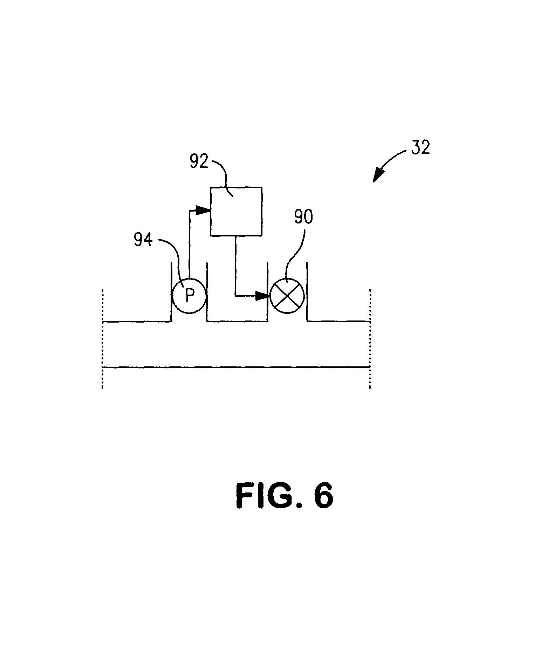

As a further option, the fluid transfer damper 32 may be an active device. For example, referring to Fig. 6, the fluid transfer damper may comprise an electrically operable valve 90, and a control circuit 92 for actively controlling the valve 90 in response to detected pressure or pressure difference. The pressure (or pressure difference) may be sensed by a pressure sensor 94. The pressure sensor 94 may be coupled to measure pressure in the vicinity of the valve 90, or at some other location in the appliance. The control circuit 92 may comprise a processor or state machine. The valve 90 may be an open-close valve, or a variable throttle valve. The control circuit 92 controls the timing and/or throat size of the valve 90. The control circuit 92 controls the valve 90 to resist to transient fluid flow, at least in the outflow direction corresponding to compression of the cuff, e.g. by delaying opening the valve under such conditions, or setting a slow flow rate. The control circuit 92 also controls the valve 90 to permit self-adjustment over time, by opening the valve in the absence of transient variation. The control circuit 92 may also control the valve 90 to open to allow the cuff to distend easily, in a similar manner to the damper arrangement of Fig. 3.

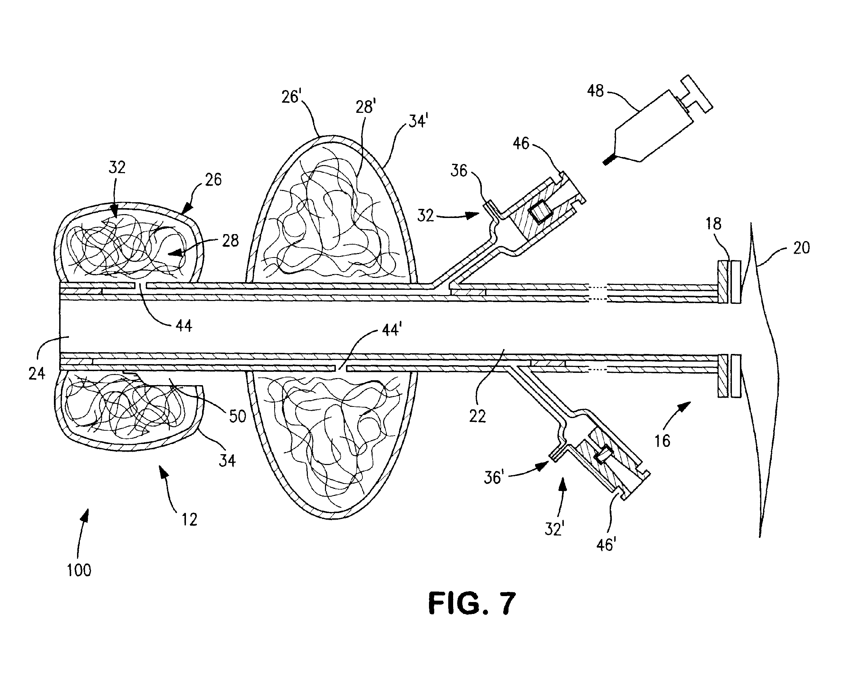

Fig. 7 illustrates a drainage appliance which is a transcecal ileostomy catheter 100 of the type described in US 20080312614 A1. This catheter 100 (also referred to as a balloon catheter) includes a distal portion having a distal resiliently deformable device 28 within the distal cuff 26 surrounded by the cuff wall 34. The distal portion is inserted within and blocks the small intestine. A proximal portion includes a proximal cuff 26' having a resiliently deformable device 28' within a cuff wall 34'. The proximal portion is inserted within and blocks the large intestine.

The proximal portion includes a separate fluid admission and withdrawal system from the distal portion. It includes a connector 45', restrictor 36', fluid transfer chamber 32', and aperture 44'.

In use this transcecal ileostomy catheter 100 is designed for the drainage of intestinal fluids. The catheter 100 is adapted to be percutaneously inserted through the abdominal wall and large intestine wall into the small intestine. The distal and proximal portions are designed to be expanded within the small intestine behind the Bauhin valve and in the large intestine, respectively. The balloon catheter allows drainage of intestinal fluids/feces such that a portion of the intestinal tract after resection is bypassed.

It will be appreciated that the foregoing description is illustrative of preferred embodiments of the invention, and that many alternatives, equivalents and improvements may be included within the claims of the invention.

## Claims

1. An appliance (10) for draining body waste from the rectum or urinary tract, the appliance comprising a distal portion configured for insertion into the rectum, intestinal or urinary tract, and the appliance further comprising:
(a) a cuff (26) at the distal portion for engaging body tissue;
(b) a resiliently deformable device (28) for urging distension of the cuff (26), the resiliently deformable device (28) being configured to deform resiliently when the cuff (26) is compressed, the resilient deformation generating a return force to counter the compression and urge distension;
(c) a fluid-containing chamber (30) of variable volume depending on at least one selected from: degree of distension of the cuff (26); degree of deformation of the resiliently deformable device (28);
**characterized by**
a fluid-transfer damper (32) for restricting fluid flow with respect to the fluid-containing chamber (30) when the chamber changes size (30), at least when the change corresponds to compression of the cuff (26), wherein the fluid transfer damper (32) is disposed in a port having one side communicating with the fluid-containing chamber (30) and another side communicating with ambient atmosphere.

2. The appliance (10) of claim 1, further comprising a tube (14) defining a drainage passage and extending to the distal portion (12), the cuff (26) extending around a peripheral surface portion of the tube (14) at the distal portion (12) of the appliance (10).

3. The appliance (10) of claim 1, wherein the resiliently deformable device (28) is arranged within the fluid-containing chamber (30) or within the cuff (26) or has a closed-loop form.

4. The appliance (10) of claim 3, wherein the fluid-containing chamber (30) has a size that varies according to degree of deformation of the resiliently deformable device.

5. The appliance (10) of claim 1, wherein the resiliently deformable device (28) has a first spring modulus at an inner periphery and a second spring modulus at an outer periphery, the first spring modulus being different from the second spring modulus.

6. The appliance (10) of claim 1, wherein the resiliently deformable device (28) is selected from: a device comprising at least one foam member; a spring member.

7. The appliance (10) of claim 6, wherein the resiliently deformable device (28) comprises a plurality of foam member segments arranged sequentially in a peripheral direction or arranged one within another.

8. The appliance (10) of claim 1, wherein the fluid-containing chamber (30) is defined at least partly by the cuff (26).

9. The appliance (10) of claim 1, wherein the fluid-transfer damper (32) is disposed at a portion of the appliance that is distinct from the cuff and, in use, is outside of the body.

10. The appliance (10) of claim 1, wherein the fluid-transfer damper (32) has the structure of a partially permeable plug, membrane or other restriction for example PTFE, silicone rubber or dense polyurethane foam.

11. The appliance (10) of claim 1, wherein the resiliently deformable device (28) and the fluid-containing chamber (30) are arranged in a distension control unit that is distinct from, and coupled in pressure communication with, the cuff.

12. The appliance (10) of claim 11, wherein a fluid inflation line (74) couples the distension control unit (74) and the cuff (26) to provide said pressure communication, the fluid inflation line (74) being part of an inflation-fluid-containing region not in fluid communication with said fluid-containing chamber (30).

13. The appliance (10) of claim 11, wherein the fluid-transfer damper (32) is disposed at the distension control unit (70).

14. The appliance (10) of claim 11, wherein the degree of deformation of the resiliently deformable member is used to indicate, or to generate an indication of, fill status of the cuff.

15. The appliance (10) of claim 1, wherein the resiliently deformable device (28) is configured to be resiliently compressible with compression of the cuff (26).

## Patentansprüche

1. Eine Vorrichtung (10) zum Ableiten von Körperausscheidungen aus dem Rektum oder dem Harntrakt, wobei die Vorrichtung einen zum Einführen in das Rektum, den Darm- oder Harntrakt konzipierten distalen Teil und weiterhin die folgenden Bestandteile umfasst:
(a) eine Manschette (26) in dem distalen Teil zum Ineingriffnehmen von Körpergewebe;
(b) eine elastisch verformbare Einheit (28) zum Forcieren einer Dehnung der Manschette (26), wobei die elastisch verformbare Einheit (28) so konzipiert ist, dass sie sich elastisch verformt, wenn die Manschette (26) komprimiert wird, wobei die elastische Verformung eine Rückzugskraft entwickelt, um der Komprimierung entgegenzuwirken und eine Dehnung zu forcieren;
(c) eine flüssigkeitsenthaltende Kammer (30) mit variablem Volumen, das abhängig ist von mindestens einem Faktor, ausgewählt aus: Dehnungsgrad der Manschette (26); Verformungsgrad der elastisch verformbaren Einheit (28);
**gekennzeichnet durch**
eine Flüssigkeitstransferklappe (32) zum Begrenzen des Flüssigkeitsflusses in Bezug auf die flüssigkeitsenthaltende Kammer (30), wenn sich die Größe der Kammer ändert (30), zumindest wenn die Änderung der Komprimierung der Manschette (26) entspricht, wobei die Flüssigkeitstransferklappe (32) in einer Öffnung angeordnet ist, bei der eine Seite mit der flüssigkeitsenthaltenden Kammer (30) und die andere Seite mit der Umgebungsatmosphäre verbunden ist.

2. Vorrichtung (10) nach Anspruch 1, weiterhin eine Röhre (14) umfassend, die eine Abflussleitung definiert und sich bis zu dem distalen Teil (12) erstreckt, wobei sich die Manschette (26) um einen peripheren Oberflächenteil der Röhre (14) an dem distalen Teil (12) der Vorrichtung (10) erstreckt.

3. Vorrichtung (10) nach Anspruch 1, wobei die elastisch verformbare Einheit (28) innerhalb der flüssigkeitsenthaltenden Kammer (30) oder innerhalb der Manschette (26) angeordnet ist oder die Form eines geschlossenen Kreislaufs hat.

4. Vorrichtung (10) nach Anspruch 3, wobei die flüssigkeitsenthaltende Kammer (30) eine Größe hat, die in Abhängigkeit vom Verformungsgrad der elastisch verformbaren Einheit variiert.

5. Vorrichtung (10) nach Anspruch 1, wobei die elastisch verformbare Einheit (28) ein erstes Elastizitätsmodul in einer inneren Peripherie und ein zweites Elastizitätsmodul in einer äußeren Peripherie hat, wobei sich das erste Elastizitätsmodul von dem zweiten Elastizitätsmodul unterscheidet.

6. Vorrichtung (10) nach Anspruch 1, wobei die elastisch verformbare Einheit (28) ausgewählt wird aus: einem Element, das mindestens ein Schaumelement umfasst; einem Elastizitätselement.

7. Vorrichtung (10) nach Anspruch 6, wobei die elastisch verformbare Einheit (28) eine Vielzahl von Schaumelementsegmenten umfasst, die der Reihe nach in peripherer Richtung oder ineinander angeordnet sind.

8. Vorrichtung (10) nach Anspruch 1, wobei die flüssigkeitsenthaltende Kammer (30) zumindest teilweise von der Manschette (26) umgrenzt ist.

9. Vorrichtung (10) nach Anspruch 1, wobei die Flüssigkeitstransferklappe (32) in einem Teil der Vorrichtung angeordnet ist, der von der Manschette getrennt ist und sich bei Verwendung außerhalb des Körpers befindet.

10. Vorrichtung (10) nach Anspruch 1, wobei die Flüssigkeitstransferklappe (32) die Struktur eines teilweise durchlässigen Stopfens, einer Membran oder einer anderer Beschränkung, z. B. PTFE, Silikonkautschuk oder dichten Polyurethan-Schaum, hat.

11. Vorrichtung (10) nach Anspruch 1, wobei die elastisch verformbare Einheit (28) und die flüssigkeitsenthaltende Kammer (30) in einem Dehnungsregler angeordnet sind, der von der Manschette getrennt und bei der Druckübertragung mit der Manschette gekoppelt ist.

12. Vorrichtung (10) nach Anspruch 11, wobei eine Flüssigkeitszuleitung (74) den Dehnungsregler (74) und die Manschette (26) koppelt, um die Druckübertragung zu ermöglichen, wobei die Flüssigkeitszuleitung (74) Teil eines Zuleitungsflüssigkeit enthaltenden Bereichs ohne Flüssigkeitsübertragungen mit der flüssigkeitsenthaltenden Kammer (30) ist.

13. Vorrichtung (10) nach Anspruch 11, wobei die Flüssigkeitstransferklappe (32) an dem Dehnungsregler (70) angeordnet ist.

14. Vorrichtung (10) nach Anspruch 11, wobei der Verformungsgrad der elastisch verformbaren Einheit zum Anzeigen oder zum Erstellen einer Anzeige des Füllstands der Manschette verwendet wird.

15. Vorrichtung (10) nach Anspruch 1, wobei die elastisch verformbare Einheit (28) so konzipiert ist, dass sie beim Komprimieren der Manschette (26) elastisch komprimierbar ist.

## Revendications

1. Un appareil (10) pour le drainage de déchets corporels par le rectum ou la voie urinaire, comprenant une partie distale configurée pour l'insertion dans le rectum, l'appareil intestinal ou la voie urinaire, l'appareil comprenant également :
(a) une manchette (26) à la partie distale, pour l'engagement avec le tissu corporel;
(b) un dispositif à déformation élastique (28) pour inciter la distension de la manchette (26), le dispositif à déformation élastique (28) étant configuré pour se déformer de façon élastique lors de la compression de la manchette (26), la déformation élastique produisant une force de rappel pour compenser la compression et la distension incitée ;
(c) une chambre à fluide (30) au volume variable tributaire d'au moins un des suivants : degré de distension de la manchette (26) ; degré de déformation du dispositif à déformation élastique (28) ;
**caractérisé par**
un amortisseur de transfert de fluide (32) pour la restriction du débit de fluide relativement à la chambre à fluide (30) lors de la variation de la taille de la chambre (30), au moins lorsque la variation correspond à la compression de la manchette (26), l'amortisseur de transfert de fluide (32) étant disposé dans un orifice dont un côté est en communication avec la chambre à fluide (30), l'autre avec l'air ambiant.

2. L'appareil (10) selon la revendication 1, comprenant également un tube (14) définissant une voie de drainage, et s'étendant vers la partie distale (12), la manchette (26) s'étendant autour d'une partie de la surface périphérique du tube (14) à la partie distale (12) de l'appareil (10).

3. L'appareil (10) selon la revendication 1, dans lequel le dispositif à déformation élastique (28) est disposé au sein de la chambre à fluide (30) ou dans la manchette (26), ou se présente sous forme de circuit fermé.

4. L'appareil (10) selon la revendication 3, dans lequel la taille de la chambre à fluide (30) varie en fonction du degré de déformation du dispositif à déformation élastique.

5. L'appareil (10) selon la revendication 1, dans lequel le dispositif à déformation élastique (28) possède un premier module d'élasticité sur un pourtour interne et un deuxième module d'élasticité sur un pourtour externe, le premier module d'élasticité étant différent du deuxième module d'élasticité.

6. L'appareil (10) selon la revendication 1, dans lequel le dispositif à déformation élastique (28) est sélectionné parmi les suivants : un dispositif comprenant au minimum un élément à mousse ; un élément à ressort.

7. L'appareil (10) selon la revendication 6, dans lequel le dispositif à déformation élastique (28) comprend une série de segments à mousse disposés séquentiellement dans une direction périphérique ou disposés l'un dans l'autre.

8. L'appareil (10) selon la revendication 1, dans lequel la chambre à fluide (30) est définie au moins en partie par la manchette (26).

9. L'appareil (10) selon la revendication 1, dans lequel l'amortisseur de transfert de fluide (32) est disposé sur une partie de l'appareil qui se distingue de la manchette, et, en cours d'usage, se trouve à l'extérieur du corps.

10. L'appareil (10) selon la revendication 1, dans lequel l'amortisseur de transfert de fluide (32) présente la structure d'un obturateur, d'une membrane, ou autre restriction partiellement perméable, par exemple PTFE, caoutchouc silicone ou mousse de polyuréthane dense.

11. L'appareil (10) selon la revendication 11, dans lequel le dispositif à déformation élastique (28) et la chambre à fluide (30) sont disposés dans un dispositif de limitation de la distension, qui se distingue de la manchette et est accouplé par communication de pression avec celle-ci.

12. L'appareil (10) selon la revendication 11, dans lequel une conduite de gonflage fluide (74) raccorde le régulateur de distension (74) et la manchette (26) pour assurer ladite communication de pression, la conduite de gonflage fluide (74) faisant partie d'une zone de gonflage contenant du fluide, qui n'est pas en communication par le fluide avec ladite chambre à fluide (30).

13. L'appareil (10) selon la revendication 11, dans lequel l'amortisseur de transfert de fluide (32) est disposé au niveau du régulateur de distension (70).

14. L'appareil (10) selon la revendication 11, dans lequel le degré de déformation de l'élément à déformation élastique sert à indiquer, ou à produire l'indication de, l'état de remplissage de la manchette.

15. L'appareil (10) selon la revendication 1, dans lequel le dispositif à déformation élastique (28) est configuré pour être compressible de façon élastique avec la compression de la manchette (26).
